Europäisches Patentamt

European Patent Office

Office européen des brevets

(19)

(11) Veröffentlichungsnummer : **0 055 869**
**B1**

(12) **EUROPÄISCHE PATENTSCHRIFT**

(45) Veröffentlichungstag der Patentschrift :
30.10.85

(21) Anmeldenummer : 81110853.9

(22) Anmeldetag : 30.12.81

(51) Int. Cl.⁴ : **G 01 N 33/542**

(54) Verfahren zur Bestimmung von Liganden, Test-Kit zur Durchführung des Verfahrens und seine Verwendung.

(30) Priorität : 02.01.81 DE 3100062

(43) Veröffentlichungstag der Anmeldung :
14.07.82 Patentblatt 82/28

(45) Bekanntmachung des Hinweises auf die Patenterteilung : 30.10.85 Patentblatt 85/44

(84) Benannte Vertragsstaaten :
AT BE CH DE FR GB IT LI LU NL SE

(56) Entgegenhaltungen :
EP-A- 0 017 908
EP-A- 0 034 775
DE-A- 2 754 086
DE-A- 2 830 862
DE-A- 3 003 959
DE-A- 3 006 710
GB-A- 2 060 623
CHEMICAL ABSTRACTS, Band 89, Nr. 21, 20. November 1978, Seite 251, Nr. 175696w, Columbus, Ohio, USA D.A. MATTHEWS et al.: "Dihydrofolate reductase from Lactobacillus casei. X-ray structure · of the enzyme methotrexate NADPH complex"

(73) Patentinhaber : Thoma, Hans A., Dr.
Giselastrasse 3
D-8000 München 40 (DE)

(72) Erfinder : Thoma, Hans A., Dr.
Giselastrasse 3
D-8000 München 40 (DE)

(74) Vertreter : Hansen, Bernd, Dr.rer.nat. et al
Hoffmann, Eitle & Partner Patentanwälte Arabellastrasse 4
D-8000 München 81 (DE)

**Beschreibung**

Die Erfindung betrifft ein Verfahren zur Bestimmung von Liganden unter Verwendung von spezifisch bindendem Rezeptor und Ligand-Marker-Konjugat, Test-Kit zur Durchführung des Verfahrens sowie dessen Verwendung.

In den letzten Jahren sind zahlreiche Methoden entwickelt worden, um diagnostisch wichtige Substanzen, wie Wirkstoffe von Arzneimitteln, Drogen oder Narkotika, Hormone, Polypeptide, Stoffwechselprodukte, Toxine etc., in Körperflüssigkeiten wie Blut oder Harn, bestimmen zu können. Insbesondere in der Diagnostik besteht ein Bedürfnis nach schnell und einfach durchzuführenden, exakten Bestimmungsmethoden. Dabei ist es wesentlich, dass diese Bestimmungsmethoden äusserst empfindlich sind, da die zu bestimmenden Substanzen häufig nur in sehr geringen Konzentrationen vorliegen.

Unter anderem wurde eine Reihe von Methodiken entwickelt, die auf der Fähigkeit eines Rezeptors, im allgemeinen eines Antikörpers beruhen, mit einem Liganden einen Komplex hoher molekularer Spezifität zu bilden. Während die Spezifität dieser Techniken im wesentlichen auf der Schlüssel-Loch-Komplementarität beruht, ist die Empfindlichkeit von der Komplexbildungskonstante (thermodynamische Gleichgewichtskonstante) von Rezeptor und Ligand abhängig. Bei Verwendung eines markierten bzw. modifizierten Liganden und Durchführung einer Konkurrenzreaktion von markierten und unmarkierten Liganden um die freien Bindungsstellen, kann — sofern die Ligandenkonzentration in der Probe die einzige Variable des Testsystems darstellt — aus dem Verhältnis zwischen freien und Antikörpergebundenen markierten Liganden auf die unbekannte Konzentration des Liganden in der Probe geschlossen werden. Zur Auswertung ist es dann erforderlich, dass entweder

a) freier und Antikörper-gebundener, markierter Ligand durch geeignete Verfahren getrennt werden : z. B. heterogene Methoden, wie RIA oder ELISA, oder

b) freier und rezeptorgebundener, markierter Ligand unterschiedliche Signale ergeben.

Als Markierung für die Liganden sind eine Reihe von Stoffgruppen mit speziellen physikochemischen oder biochemischen Eigenschaften beschrieben worden. Insbesondere wurden die Liganden mit Markierungssubstanzen, wie Radioisotopen, Enzymen, Phagen, fluoreszierenden Substanzen und freien Radikalen umgesetzt.

Diese Methoden nach dem Stand der Technik weisen jedoch zahlreiche Nachteile auf. So sind z. B. bei den radioimmunologischen Methoden die Gefahren der Handhabung radioaktiv-markierter Substanzen und die entsprechenden behördlichen Auflagen hinderlich. Der Einsatz fluoreszierender Gruppen ist aufgrund sich ergebender Verstärkungsprobleme begrenzt. Andere Verfahren sind in ihrer Durchführung zu aufwendig oder weisen das Problem mangelnder Sensibilität oder zu geringer Spezifität auf.

Die Nachteile der Verfahren nach dem Stand der Technik werden mittels der vorliegenden Erfindung dadurch überwunden, dass bei der analytischen Bestimmung ein nicht-markiertes bzw. nicht-modifiziertes Molekül als Signalmolekül verwendet wird.

Der Erfindung liegt die Aufgabe zugrunde, eine neue Bestimmungsmethode zur Verfügung zu stellen. Hiernach soll es insbesondere möglich sein, durch Bildung eines stabilen ternären Komplexes ein nicht-modifiziertes Signalmolekül zu erhalten, wodurch die bestehenden Analysenmethoden zum Nachweis von Haptenen, Antigenen, etc. bereichert und verbessert werden sollen. Insbesondere soll das neue Verfahren eine hohe Ansprechbarkeit im p-Mol-Bereich gewährleisten, sowie mit hoher Genauigkeit möglichst störungsfrei auf homogene Weise durchzuführen sein.

Die erfindungsgemässe Aufgabe wird dadurch gelöst, dass ein Verfahren zur Bestimmung von Liganden unter Verwendung von spezifisch bindendem Rezeptor und Ligand-Marker-Konjugat zur Verfügung gestellt wird, das die folgenden Schritte umfasst :

a) ein den Liganden spezifisch bindender Rezeptor wird zur Testprobe zugegeben ;

b) ein Konjugat, bestehend aus einem Marker und dem Liganden wird zur Testprobe gegeben ;

c) Signalmolekül und eine weitere Komponente werden zu der genannten Testprobe zugegeben, um mit dem nicht-gebundenen Ligand-Marker-Konjugat einen ternären Komplex zu bilden ; und

d) freies Signalmolekül wird bestimmt.

Im weiteren soll durch die Erfindung ein Test-Kit : zur Verfügung gestellt werden, der die routinemässige Bestimmung eines Liganden ermöglicht, wobei dieser Test-Kit im Gemisch oder voneinander getrennt eine spezifisch bindenden Rezeptor, ein Ligand-Marker-Konjugat, Signalsubstanz sowie die zur Bildung eines ternären Komplexes erforderliche weitere Komponente und die Substanzen des Nachweissystems zur Bestimmung von freier Signalsubstanz enthält.

Mit der Bezeichnung « Ligand » sollen hier grundsätzlich all diejenigen Liganden erfasst sein, die in der DE-AS-2 223 385 in den Spalten 22 bis 46 beschrieben worden sind, worauf ausdrücklich für die Zwecke der vorliegenden Erfindung Bezug genommen wird. Der Ligand kann in seinem Molekulargewicht stark variieren, von etwa 100 bis 1 000 000. Dieser Molekulargewichtsbereich wird durch das erfindungsgemässe Verfahren erfasst, vorausgesetzt, es steht ein Rezeptor zur Verfügung, der den Liganden spezifisch bindet.

Lediglich zur Erläuterung sei hier angeführt, dass es sich hierbei u. a. um Antigene, um Heptene oder auch um natürlich vorkommende Rezeptoren handeln kann. Unter diese Gruppe fallen eine Fülle biologisch aktiver Substanzen, wie Hormone, z. B. Thyroxin, viele Steroide, wie Östrogene, Cortison, Corticosteron oder Östradiol, Peptide, Polypeptide, wie Insulin, Lipoproteine, Glycoproteine, Sterine, Nukleinsäuren, Mono- und Polysaccharide, Alkaloide, Vitamine, Enzyme, Coenzyme, aber auch Wirkstoffe von Arzneimitteln, häufig eingenommene Drogen, wie Benzdiazepine, Antibiotika, sowie Toxine, Pestizide, celluläre oder extracelluläre Gewebekomponenten und aus Menschen oder Tieren isolierte Antikörper.

Als Komponenten, die mit Hilfe des erfindungsgemässen Verfahrens bestimmt werden können, sind insbesondere zu nennen : Tumor-Antigene, Rheumatoid-Faktor, Myoglobin, Ferritin, Analgetika, wie Morphin, Amphetamin, Gentamycin, Barbiturate, sowie Pharmaka, wie Digoxin, Digitoxin, Phenobarbital, Diphenylhydantoin, sowie auch Vitamine, Nahrungsmittelschadstoffe, Herbicide, Insekticide und Aflatoxine.

Ausserdem ist das erfindungsgemässe Verfahren anwendbar zur Bestimmung von TSH, HCG, IgG und Hepatitis-Antigen.

Bei der Bezeichnung « Rezeptor » handelt es sich um spezifisch bindende Moleküle, insbesondere Antikörper sowie spezifisch bindenden Proteine, wie z. B. Transportproteine. Die Gewinnung der Antikörper erfolgt beispielsweise durch Immunisierung von Tieren mit entsprechend hochgereinigtem Antigen oder Carrier-gebundenem Hapten. Die Antikörper sind häufig Gammaglobuline, die mit hoher Spezifität den Liganden zu binden vermögen. Unter der Bezeichnung Rezeptor sollen hier neben normalen Antikörpern auch sogenannte Anti-Inhibitor-Antikörper, gegebenenfalls in Kombination mit Anti-IgG-Antikörpern und/oder auch Fab-Fragmente verstanden werden.

Der Begriff « Ligand-Marker-Konjugat » in der vorliegenden Beschreibung bezieht sich auf einen Liganden, ein Analogon eines Liganden oder dessen Teil, der mit einer Substanz markiert ist, welche an der Bildung eines ternären Komplexes teilnimmt. An jeweils einem Liganden kann ein oder mehr als ein Markierungsmolekül gebunden sein. Das Ligand-Marker-Konjugat wird im allgemeinen nach Methoden erzeugt, wie sie für die Bindung anderer Markersubstanzen, wie z. B. Enzyme oder Coenzyme, Verwendung finden. Die Kopplungsreaktion hängt im wesentlichen von den am Ligand bzw. am Markermolekül zur Verfügung stehenden bzw. einführbaren reaktiven bzw. aktivierbaren Gruppen ab. Im allgemeinen werden die bekannten Methoden der Peptidchemie, der Affinitätschromatografie, der Solidphase-Technologie, analog angewandt. Mit Vorteil kann man z. B. bei kleinen Haptenen analoge Reaktionen zur Synthese des Antigens, z. B. Kopplung an Albumin oder Thyroglobulin verwenden. Vorteilhaft sind auch Kopplungsverfahren mit bifunktionellen Reagentien, wie z. B. Carbodiimiden (z. B. 1-Cyclohexyl-3-(morpholinoethyl)-carbodiimid), Glutaraldehyd oder m-Maleinimidobenzyl-N-hydroxysuccinimidester, sowie spezielle Verfahren, wie die reduktive Aminierung, gemischte Anhydridmethoden und Thioetherverfahren.

Wesentlich ist bei der Bildung des Ligand-Marker-Konjugats, dass auch nach Durchführung der Kopplungsreaktion die Markerkomponente im Konjugat zur Teilnahme an der Bildung des ternären Komplexes befähigt bleibt und nicht etwa durch den Ligandanteil im Konjugat eine sterische Hinderung erfährt.

Die Markerkomponente bildet erfindungsgemäß mit einem Signalmolekül und einer weiteren Komponente, vorzugsweise einem Eiweissmolekül, einen stabilen ternären Komplex. Bei konstanter Menge an zur Probe zugegebenem Ligand-Marker-Konjugat und Signalmolekül kann dann über die zu bestimmende Menge an freiem Signalmolekül auf die Menge an gebundenem Signalmolekül umgerechnet werden. Die Menge an gebundenem Signalmolekül entspricht wiederum der Menge an im ternären Komplex gebundenem Ligand-Marker-Konjugat, so dass über diese Grösse auf das am Rezeptor gebundene Ligand-Marker-Konjugat bzw. auf den am Rezeptor gebundenen Liganden umgerechnet werden kann.

Als Markierungssubstanzen, die sich für die Bildung des Ligand-Marker-Konjugates besonders eignen, sind zu nennen : Enzyme, Coenzymen, Chelatbildner, Proteine, Peptide und Polypeptide. Auf besonders vorteilhafte Marker wird bei der Diskussion der ternären Komplexe noch im einzelnen eingegangen.

Unter dem « Signalmolekül » wird gemäss der Erfindung eine Substanz verstanden, die an der Bildung eines stabilen, ternären Komplexes teilnimmt und welche unmittelbar durch ein beliebiges analytisches Verfahren bestimmt wird. Ein wesentlicher Vorteil gemäss dem erfindungsgemässen Verfahren ist dabei, dass diese Signalsubstanz ein nicht-markiertes bzw. nicht-modifiziertes Molekül darstellt.

Als Signalsubstanzen eignen sich insbesondere Coenzyme, wie NADPH, Enzyme, Metalle, insbesondere Übergangsmetalle, u. a. Europium, sowie prinzipiell sämtliche, einen stabilen, ternären Komplex bildenden Komponenten, welche mit Hilfe eines geeigneten Nachweisverfahrens schnell und genau bestimmt werden können.

Als Bestimmungsmethode eignet sich jede Nachweismethode, die eine präzise und einfache Bestimmung der Menge an freiem Signalmolekül erlaubt, so u. a. die Methode des Recycling, der Biolumineszenz, der Fluoreszenzmessung, Elektronenspin-Messung und der enzymatischen Messung.

Die sogenannte « weitere Komponente » innerhalb des ternären Komplexes kann eine beliebige niedermolekulare oder hochmolekulare Substanz darstellen, die zusammen mit dem Markermolekül und dem Signalmolekül einen stabilen, ternären Komplex bildet. Insbesondere kann diese weitere Kompo-

**0 055 869**

nente ein Proteinmolekül darstellen, vorzugsweise ein Enzym oder einen Antikörper.

Als « ternärer Komplex » wird gemäss der Erfindung ein Komplex, bestehend aus dem Markermolekül, dem Signalmolekül und der weiteren Komponente, vorzugsweise einem Protein, verstanden.

Durch das Vorliegen eines stabilen ternären Komplexes sowie durch die sterische Hinderung des Antikörper gebundenen Ligand-Marker-Konjugates zur Einbeziehung in den entsprechenden ternären Komplex wird gewährleistet, dass gebundenes und freies Signalmolekül ein unterschiedliches Signal ergeben, wodurch deren jeweilige Bestimmung ermöglicht wird.

Als besonders vorteilhafte stabile Komplexe, die bei der Durchführung des erfindungsgemässen Verfahrens Verwendung finden können, sind u. a. zu nennen : Methotrexat/NADPH/Dihydrofolsäure reduktase, beschrieben z. B. von D.A. Matthews et al in Journal of Biological Chemistry, Band 253, 1978, Seiten 6946-6954 ; Thymidylatsynthetase/5-Fluoro-desoxyuridylat/5,10-Methylentetrahydrofolat, beschrieben z. B. von W. Rhode et al in Journal of Biological Chemistry, Band 254, 1979, Seiten 11538-11543 ; Antikörper/Chelat/Übergangsmetall ; Antikörper + Addukt aus Charge-Transfer-Komplex, bestehend aus RNA-DNA.

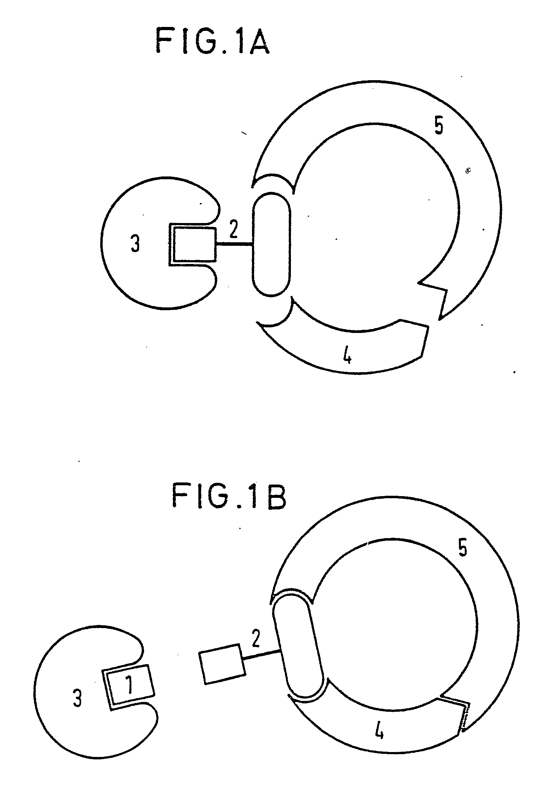

In Fig. 1A ist schematisch die Bindung des Ligand-Marker-Komplexes an den spezifischen Rezeptor dargestellt. In diesem Fall kann aufgrund sterischer Hinderung die im Konjugat gebundene Marker-Komponente nicht an der Bildung des ternären Komplexes teilnehmen, so dass auch das korrespondierende Signalmolekül frei ist und als solches der analytischen Bestimmung zugänglich ist.

In Fig. 1B ist der Fall dargestellt, dass der zu bestimmende Ligand die Bindungsstelle am Rezeptor belegt. Das Ligand-Marker-Konjugat ist frei zur Bildung des ternären Komplexes, in welchem auch das entsprechende Signalmolekül gebunden ist. Das gebundene Signalmolekül ergibt ein Signal, welches sich von dem des freien Signalmoleküls unterscheidet.

In den Fig. 1A und 1B bedeutet (1) den zu bestimmenden Liganden, (2) das Ligand-Marker-Konjugat, (3) das spezifische Rezeptormolekül, (4) das Signalmolekül und (5) die weitere Komponente des ternären Komplexes.

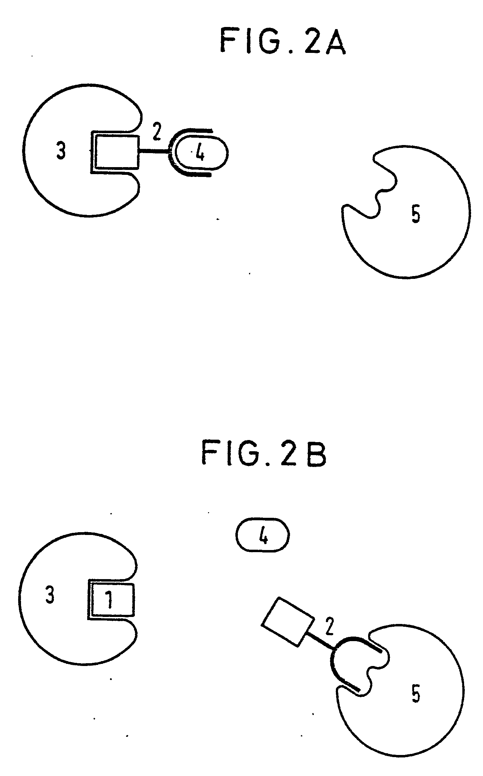

Nach einer weiteren Ausführungsform gemäss der Erfindung ist es möglich, einen ternären Komplex, bestehend aus Ligand-Marker-Konjugat, wobei das Markermolekül vorzugsweise einen Chelatbildner darstellt, sowie einem von diesem Marker gebundenen Übergangsmetall zu bilden. Durch Zusatz eines zweiten Antikörpers, der gegen das Ligand-Marker-Konjugat gerichtet ist, wird dieses, sofern es im nicht-gebundenen Zustand vorliegt, gebunden. Zugegegenes Übergangsmetall, z. B. Europium, wird dann nur durch Reaktion im nicht-blockierten Chelatkonjugat am Rezeptor-Komplex (Fig. 2A) gebunden und in seiner physikalischen Eigenschaft verändert. Auch in diesem Fall resultiert das Signal aus einem nichtmodifizierten Molekül. Die Menge an freiem Übergangsmetall kann durch beliebige Methoden bestimmt werden, so z. B. im Falle der Verwendung von Europium als Übergangsmetall durch Fluoreszenzmessungen.

Diese Ausführungsform des erfindungsgemässen Verfahrens ist in Fig. 2A und 2B dargestellt, wobei (1) den zu bestimmenden Liganden, (2) das Ligand-Marker-Konjugat, (3) den spezifisch bindenden Rezeptor, (4) das Übergangsmetall und (5) den gegen das Ligand-Marker-Konjugat gerichteten Antikörper darstellen.

Das erfindungsgemässe Verfahren wird nachstehend anhand von einigen nicht optimierten Versuchsbeispielen zur Herstellung des Konjugates und zur Ermittlung von entsprechenden Eichkurven erläutert. Hierbei können die Versuchsbedingungen zur Herstellung des Konjugates, zur Reaktion mit Rezeptor etc., in der dem Fachmann bekannten Art und Weise je nach Wahl des speziellen System Ligand-Marker/ternärer Komplex gewählt werden.

Im allgemeinen ergeben sich die folgenden Reaktionszeiten für die einzelnen Schritte :

a) bei Haptenen. Reaktionszeit Antikörper/Hapten : 30 s bis 30 h ; Reaktionszeit zur Bildung des ternären Komplexes : 0 min bis 60 min.

b) für grössere Liganden, z. B. Antigene : Reaktionszeit Antikörper/Antigen : 3 min bis 48 h ; Reaktionszeit zur Bildung des ternären Komplexes 0 min bis 60 min.

Das Verfahren zur Bestimmung eines Liganden, z. B. eines Haptens oder Antigens, in einer biologischen Probe kann sich durch folgende Schritte auszeichnen :

a) Der Probe wird eine bestimmte Menge (Überschuss) eines Antikörpers zugesetzt, der gegenüber dem Ligand in der Probe spezifisch ist, um einen Ligand-Antikörper-Komplex zu bilden.

b) Dem unter a) gebildeten Gemisch wird eine bekannte Menge einer Lösung des Ligand-Marker-Konjugates zugesetzt, und zwar möglichst mit einem Überschuss gegenüber der Menge, die erforderlich ist, um die Menge des nicht-gebundenen Rezeptors bzw. Antikörpers zu komplexieren.

c) Zu dem Gemisch von b) erfolgt die Zugabe einer bekannten Menge an Signalmolekül, wobei diese Menge im Überschuss gegenüber dem nicht-rezeptorgebundenen Anteil an Ligand-Marker-Konjugat vorliegt. Ausserdem wird zu diesem Gemisch von b) die weitere, zur Bildung des ternären Komplexes erforderliche Komponente zugegeben.

d) In dem Gemisch c) wird die Menge des nicht-gebundenen Signalmoleküls durch geeignete analytische Methoden, wie z. B. Lumineszenzmessung, Fluoreszenzmessung Enzymaktivitätsmessung oder Recycling-Methode, bestimmt.

Das obige Verfahren kann in der angegebenen Reihenfolge, jedoch auch in andersartigen Anordnungen, wie sie dem Fachmann geläufig sind, durchgeführt werden.

4

Einer der Hauptvorteile dieses Verfahrens liegt darin, dass eine Trennung gebundener und freier Anteile an Signalmolekül nicht erforderlich ist. Dies schliesst jedoch die Anwendung einer solchen Trennstufe bei einem Test nach der Inkubation nicht aus. Eine Trennung der Anteile oder Fraktionen kann zuweilen wünschenswert sein, um Substanzen in der Probe zu entfernen, die die Bildung des ternären Komplexes oder die Bestimmung des freien Signalmoleküls stören können. Die Trennung kann unter Anwendung irgendeiner der vielen für den Radioimmuntest beschriebenen Techniken, einschliesslich Gelfiltration, Adsorption und Ionenaustauschchromatografie, fraktionierter Fällung, Solid-Phase-Trennung und Elektrophorese durchgeführt werden.

1. Synthese von Testosteron-Methotrexat-Konjugat (Methode nach Erlanger)

0,1 mmol MTX (Methotrexat) wurden in 1 500 μl DMF und 40 μl tri-n-Butylamin gegeben. Bei ca. 10 °C wurde 0,1 mmol Chlorameisensäureester hinzugegeben. Das Reaktionsgemisch wurde 20 Minuten bei 4 °C stehen gelassen.

Daraufhin wurden 30 mg AAO (3-Amino-5α-dihydroandrostan-17β-ol) zugegeben. Nachdem weitgehende Auflösung erfolgte, wurden 210 μl 1N NaOH zugesetzt. Das Reaktionsgemisch wurde 1 h bei Raumtemperatur stehen gelassen. Daraufhin wurden 100 μl 1N NaOH zugegeben und wieder 1 h bei Raumtemperatur stehen gelassen. Das Gemisch wurde dann durch Glasfasern in der Pipette filtriert ; zum Filtrat wurden 3 ml Wasser zugegeben. Es bildete sich ein Niederschlag. Dieser wurde am nächsten Tag abzentrifugiert und 20 ml Ethanol zugegeben, worauf sich der Niederschlag in der Wärme auflöste.

2. Nachweis der Bildung eines ternären Komplexes

Die Bildung eines ternären Komplexes wurde nachgewiesen durch die Abhängigkeit der Hemmung von der Menge des zugegebenen Testosteron-Methotrexat-Konjugates zu Dihydrofolsäurereduktase. In der nachstehenden Tabelle sind die Versuche mit unterschiedlichen Mengen an Testosteron-Methotrexat-Konjugat zusammengestellt. Die Extinktionswerte zeigen deutlich eine Abnahme der Enzymaktivität mit zunehmender Konjugatkonzentration.

Tabelle I

| Probe | Testosteron-Methotrexat-Konjugat μl | NADPH μl | DHF-Reduktase ul | Substrat μl | Enzym μl | Extinktion |
|---|---|---|---|---|---|---|
| 1 | 0 | 50 | 200 | 250 | 50 | 0,114 |
| 2 | 10 | 50 | 200 | 250 | 50 | 0,104 |
| 3 | 50 | 50 | 200 | 250 | 50 | 0,086 |
| 4 | 100 | 50 | 200 | 250 | 50 | 0,072 |
| 5 | 200 | 50 | 200 | 250 | 50 | 0,058 |

Durch Zugabe von entsprechenden Pufferlösungen wurde das Gesamtvolumen der Proben konstant gehalten.

3. Durchführung des erfindungsgemässen Verfahrens am System DHF-Reduktase/Methotrexat/NADPH

Unter Verwendung des unter 1. hergestellten Testosteron-Methotrexat-Konjugates wurden dem Probengemisch verschiedene Mengen Testosteron sowie eine konstante Menge an Testeron-Antikörper zugegeben. Zur Bindung des freien Testosteron-Methotrexat-Konjugates in einem ternären Komplex wurden den Proben konstante Mengen an DHF-Reduktase und NADPH zugegeben. Die Bestimmung von freiem NADPH erfolgte mit Recycling-Techniken. In der nachstehenden Tabelle II sind diese Versuche im einzelnen dargestellt.

Die Auswertung der Enzymaktivität zeigt deutlich eine Verminderung bei gleichzeitiger Zugabe von Testosteron und Testosteron-Methotrexat-Konjugat.

(Siehe Tabelle II Seite 6 f.)

Tabelle II

| Probe | Puffer | Anti-körper | Testo-steron | Testosteron-Methotrexat-Konjugat | NADPH | DHF-Reduk-tase | Enzymaktivität Milliextinkt. pro min (Recycling) |
|---|---|---|---|---|---|---|---|
| 1 | + | + | − | − | + | + | 100 |
| 2 | + | + | − | + | + | + | 90 |
| 3 | + | + | + | − | + | + | 100 |
| 4 | + | + | + | + | + | + | 54 |

**Patentansprüche**

1. Kompetitives Verfahren zur Bestimmung von Liganden in einer Testprobe, wobei :
a) ein den Liganden spezifisch bindender Rezeptor zur Testprobe zugegeben wird ;
b) ein Konjugat, bestehend aus einem Marker und dem Liganden, zur Testprobe gegeben wird ; dadurch gekennzeichnet, dass
c) ein Signalmolekül und eine weitere Komponente zu der genannten Testprobe zugegeben werden, um mit dem nicht-gebundenen Ligand-Marker-Konjugat einen ternären Komplex zu bilden ; und
d) freies Signalmolekül bestimmt wird.

2. Verfahren gemäss Anspruch 1, dadurch gekennzeichnet, dass die Verfahrensschritte a) bis d) ohne zwischenzeitliche Trennstufe auf homogene Weise durchgeführt werden.

3. Verfahren nach Anspruch 1, dadurch gekennzeichnet, dass man als Ligand ein Antigen, Hapten, Hormon, Vitamin, einen pharmakologischen Wirkstoff oder einen Rezeptor einsetzt.

4. Verfahren nach Ansprüchen 1 bis 3, dadurch gekennzeichnet, dass man als Rezeptor einen Antikörper, einen Anti-Inhibitor-Antikörper, gegebenenfalls in Kombination mit Anti-IgG-Antikörpern, und/oder Fab-Fragmenten verwendet.

5. Verfahren nach Ansprüchen 1 bis 4, dadurch gekennzeichnet, dass als Markerkomponente in dem Ligand-Marker-Konjugat ein Chelatbildner, ein Vitamin oder ein Protein verwendet wird.

6. Verfahren nach Ansprüchen 1-4, dadurch gekennzeichnet, dass als Marker-Komponente Methotrexat sowie dessen Analoge dienen.

7. Verfahren nach einem oder mehreren der vorhergehenden Ansprüche, dadurch gekennzeichnet, dass als Signalmolekül ein Coenzym, ein Enzym oder ein Metall dient.

8. Verfahren nach Anspruch 7, dadurch gekennzeichnet, dass NADPH als Signalmolekül verwendet wird.

9. Verfahren nach Anspruch 7, dadurch gekennzeichnet, dass Europium als Signalmolekül dient.

10. Verfahren nach einem oder mehreren der vorhergehenden Ansprüche, dadurch gekennzeichnet, dass als ternärer Komplex das System DHF-Reduktase/Methotrexat/NADPH verwendet wird.

11. Verfahren nach einem oder mehreren der vorhergehenden Ansprüche, dadurch gekennzeichnet, dass als ternärer Komplex das System Thymidylatsynthetase/5-Fluoro-2'-desoxyuridylat/5,10-Methylentetrahydrofolat verwendet wird.

12. Verfahren nach einem oder mehreren der vorhergehenden Ansprüche, dadurch gekennzeichnet, dass als ternärer Komplex ein System aus Antikörper und einem Komplex-Addukt aus zwei Molekülen dient.

13. Verfahren nach einem oder mehreren der vorhergehenden Ansprüche, dadurch gekennzeichnet, dass als ternärer Komplex ein System Antikörper/Ligand-Chelat-Konjugat/Übergangsmetall dient.

14. Verwendung eines Ligand-Marker-Konjugates zur Durchführung einer Verdrängungsanalyse gemäss Anspruch 1 zur Bestimmung eines Liganden, wobei der Markerteil des nicht rezeptorgebundenen Konjugates an der Bildung eines ternärer Komplexes teilnimmt.

15. Test-Kit zur Durchführung des Verfahrens gemäss Ansprüchen 1 bis 13, dadurch gekennzeichnet, dass dieser im Gemisch oder voneinander getrennt einen spezifisch bindenden Rezeptor, ein Ligand-Marker-Konjugat, Signalsubstanz sowie die zur Bildung eines ternären Komplexes erforderliche weitere Komponente und die Substanzen des Nachweissystems zur Bestimmung von freier Signalsubstanz enthält.

**Claims**

1. A competitive method for the determination of ligands in a test sample, in which method :
a) a receptor capable of binding specifically with a ligand is added to the test sample ;

b) a conjugate consisting of a marker and the ligand is added to the test sample ; characterized in that

c) a signal molecule and a further component are added to the test sample in order to form a ternary complex with unbound ligand-marker conjugate ; and

d) the unbound signal molecule is determined.

2. A method according to claim 1 in which process steps a) to d) are carried out in a homogeneous manner without any intermediate separation step.

3. A method according to claim 1, characterized in that the ligand represents an antigen, hapten, hormone, vitamin, a pharmacologically active component or a receptor.

4. A method according to claims 1 to 3, characterized in that as a receptor is used an antibody, an anti-inhibitor-antibody, optionally in combination with an anti-IgG-antibody, and/or Fab-fragments.

5. A method according to claims 1 to 4, characterized in that as marker component in the ligand-marker conjugate is used a chelating agent, vitamin, or a protein.

6. A method according to claims 1 to 4, characterized in that methotrexate as well as its analogs serve as marker component.

7. A method according to one or more of the preceding claims, characterized in that a coenzyme, an enzyme or a metal serve as a signal molecule.

8. A method according to claim 7, characterized in that NADPH is used as a signal molecule.

9. A method according to claim 7, characterized in that europium is used as a signal molecule.

10. A method according to one or more of the preceding claims, characterized in that as a ternary complex is used the system DHF-reductase/methotrexate/NADPH.

11. A method according to one or more of the preceding claims, characterized in that as a ternary complex is used the system thymidylate synthetase/5-fluoro-2'-deoxyuridylate/5,10-methylene-tetra-hy-drofolate.

12. A method according to one or more of the preceding claims, characterized in that a system of antibody and a complex adduct of two molecules serves as a ternary complex.

13. A method according to one or more of the preceding claims, characterized in that a system of antibody/ligand-chelate conjugate/transition metal serves as a ternary complex.

14. The use of a ligand-marker conjugate for carrying out a displacement analysis according to claim 1 for the determination of a ligand, wherein the marker component of the non-receptor-bound conjugate takes part in the formation of a ternary complex.

15. A test-kit for carrying out the method according to claims 1 to 13, characterized in that this comprises, in a mixture or separately divided, a specifically binding receptor, a ligand-marker conjugate, a signal substance as well as a further component necessary for the formation of the ternary complex, and the components of the analytical system for the determination of free signal substance.

**Revendications**

1. Procédé compétitif pour la détermination de ligands présents dans un échantillon d'essai, dans lequel :

a) on ajoute à l'échantillon d'essai un récepteur se combinant spécifiquement au ligand ;

b) on ajoute à l'échantillon d'essai un conjugat constitué par un marqueur et le ligand ; caractérisé par le fait que

c) on ajoute à l'échantillon d'essai une molécule signal et un autre composant dans le but de former un complexe ternaire avec le conjugat-ligand-marqueur non combiné ; et

d) on détermine une molécule signal libre.

2. Procédé selon la revendication 1, caractérisé par le fait que les étapes a) à d) du procédé sont effectuées de manière homogène sans étape de séparation intermédiaire.

3. Procédé selon la revendication 1, caractérisé par le fait que l'on utilise comme ligand un antigène, un haptène, une hormone, une vitamine, une substance pharmacologiquement active ou un récepteur.

4. Procédé selon les revendications 1 à 3, caractérisé par le fait que l'on utilise comme récepteur un anticorps, un anticorps anti-inhibiteur, en combinaison, le cas échéant, avec des anticorps anti-IgG, et/ou des fragments Fab.

5. Procédé selon les revendications 1 à 4, caractérisé par le fait que l'on utilise comme composants marqueurs à l'intérieur du conjugat-ligand-marqueur un agent chélatant, une vitamine ou une protéine.

6. Procédé selon les revendications 1 à 4, caractérisé par le fait que l'on utilise comme composants-marqueurs, du méthotrexate ainsi que ses composés analogues.

7. Procédé selon l'une quelconque des revendications précédentes, caractérisé par le fait que l'on utilise comme molécule-signal un co-enzyme, un enzyme ou un métal.

8. Procédé selon la revendication 7, caractérisé par le fait que l'on utilise, comme molécule-signal, du NADPH.

9. Procédé selon la revendication 7, caractérisé par le fait que l'on utilise comme molécule-signal de l'Europium.

10. Procédé selon l'une quelconque des revendications précédentes, caractérisé par le fait que l'on utilise comme complexes ternaires le système DHF-réductase/méthotrexate/NADPH.

11. Procédé selon l'une quelconque des revendications précédentes, caractérisé par le fait que l'on utilise comme complexe ternaire le système thymidylate-synthétase/5-fluoro-2'-désoxyuridylate/5,10-méthylènetétrahydrofolate.

12. Procédé selon l'une quelconque des revendications précédentes, caractérisé par le fait que l'on utilise, comme complexe ternaire, un système constitué d'anticorps et d'une addition complexe de deux molécules.

13. Procédé selon l'une quelconque des revendications précédentes, caractérisé par le fait que l'on utilise, comme complexe ternaire, un système constitué par des anticorps/un ligand-agent chélatant/un métal de transition.

14. Utilisation d'un conjugat-ligand-marqueur pour effectuer une analyse selon la revendication 1, dans le but de déterminer un ligand, l'élément marqueur conjugat non combiné au récepteur participant à la formation d'un complexe ternaire.

15. Kit de test pour la mise en œuvre du procédé selon les revendications 1 à 13, caractérisé par le fait que ledit kit contient, en mélange ou séparément, un récepteur à combinaison spécifique, un conjugat-ligand-marqueur, une substance signal, ainsi que la composante ultérieurement requise pour la formation d'un complexe ternaire et les substances du système de démonstration dans le but de définir une substance signal libre.

0 055 869

# FIG.1A

# FIG.1B

# FIG. 2A

# FIG. 2 B